# EUROPEAN PATENT SPECIFICATION

(11) **EP 1 601 284 B1**
(45) Date of publication and mention of the grant of the patent: **15.07.2009**
(21) Application number: 04715394.5
(22) Date of filing: 27.02.2004
(51) Int. Cl.: A61B 5/00, A61M 16/04

(54) **DILATION DEVICE**
DILATATIONSVORRICHTUNG
DISPOSITIF DE DILATATION

(30) Priority: 27.02.2003 GB 0304439
(43) Date of publication of application: 07.12.2005
(73) Proprietor: Smart Surgical Appliances Ltd, London SW7 2AZ (GB)
(72) Inventor: Darzi, Ara, c/o Imperial College Innovations Ltd, Imperial College, London SW7 2BP (GB); Edwards, L., c/o Imperial College Innovations Ltd, Imperial College, London SW7 2BP (GB)
(74) Representative: Johnstone, Helen Margaret
(86) International application number: PCT/GB2004/000818
(87) International publication number: WO 2004/075743

(56) References cited:
- EP-A- 0 138 089
- EP-A- 0 594 335
- WO-A-01/49359
- DE-U- 9 416 013
- US-A- 4 693 243

## Description

This invention relates to a device for measuring pressure within a tubular anatomy, and particularly, but not exclusively, to a device for measuring pressure within the oesophagus of a human or animal.

The oesophagus is a tube that connects the mouth with the stomach. The walls of the oesophagus are very muscular, and contract rhythmically. This rhythmical movement is known as peristalsis and serves to transfer food from the mouth to the stomach for digestion within the stomach.

The oesophagus is connected to the stomach by a valve known as the lower oesophageal sphincter. The sphincter prevents the backward flow of food from the stomach into the oesophagus.

It can sometimes be necessary to measure how well the muscles of the oesophagus are working and to measure the strength of the lower oesophageal sphincter. Such measurements may assist diagnosis of a medical condition present in a patient.

These measurements are known as oesophageal manometry or as an oesophageal function or oesophageal motility study.

It is known to carry out such measurements by passing a soft tube through the nose or mouth of a patient. The tube has pressure sensors along its length and when in place can measure the pressure that is produced by the oesophageal muscles when relaxing or compressing during the peristaltic process, or the pressure within a stricture.

It is known that strictures may develop in the oesophagus. The presence of one or more strictures in an oesophagus may lead to problems and clinical conditions such as dyspepsia, dysphasia and asthma. It is therefore desirable to be able to dilate the oesophagus in the region of the stricture in order to reduce or overcome these problems.

It is known to use a device which is generally olive shaped and made of metal to dilate the oesophagus in the region of a stricture. US 3 517 128 discloses a similar dilator in the form of an expansible cage formed by a series of flexible steel ribs which, in use, bow outwards to form a baloon-like structure. These devices are available in a range of sizes, and are passed through the stricture in turn, starting with the largest device that can just pass through the stricture, and increasing the size of the device until the desired dilation of the stricture is achieved. It is also possible to use, in a similar way, soft, flexible rubber tubes in place of the metal olive shaped devices.

Surgery of the oesophagus, particularly conventional or laparoscopic fundoplication used in the treatment of gastroesophageal reflux disease can result in severe post-operative dysphagia. This is because the wrap (i.e the stomach wall wrapped around the oesophagus) is too tight. The ability to perform manometry in the oesophagus pre, intra and post operation would reduce the extent of dysphagia suffered since it would enable the wrap tension to be measured.

In addition, a means for carrying out pre, intra and post operative manometry combined with a means for measuring the dilation of strictures would assist in the selection of the most appropriate technique to use to carry out the surgery.

According to the invention there is provided a device for causing dilation of, and for measuring the pressure exerted by, a tubular anatomy comprising :
an active elements including;
a plurality of elongate flexible members, each having a first end and a second end;
a first connector connected to the first ends of the elongate flexible members;
a second connector connected to the second ends of the elongate flexible members; and
a compressor for causing one of the connectors to move towards the other connector thereby causing the elongate flexible members to bow laterally,
   characterised in that the first connector includes at least one pressure sensor to measure the pressure exerted on the active element by the tubular anatomy.

The compressor is controlled to exert a predetermined pressure on the elongate members. This means that an appropriate degree of dilation may be achieved. The pressure applied by the compressor may be either constant or stepped to allow graduated expansion to predetermined dilation

By means of the present invention, therefore, it is possible to achieve controlled dilation of a stricture in a tubular anatomy without having to use a plurality of devices each having a predetermined size. This in turn means that devices do not have to be taken in and out of the tubular anatomy, since once the device has been inserted, it can remain in place until the surgical operation or procedure is complete. This reduces the severity of dysphagia experienced after the operation or procedure.

The ability to measure pressure exerted on the active element means that when the device is in place in a tubular anatomy, the at least one pressure sensor is able to measure either the proximal or the distal pressure exerted on the active element by the walls of the tubular anatomy.

The presence of the at least one pressure sensor means the device according to the invention is able to cause dilation of a stricture in a tubular anatomy and also carry out oesophageal manometry. This means that it is not necessary to insert a separate manometer into the tubular anatomy, further reducing the severity of any dysphagia experienced by the patient.

Advantageously, the device further comprises at least one stop for preventing over-compression of the active element This ensures that over dilation does not occur. The expansion of the active element is restricted by the at least one stop, past which the moveable element cannot move. In other words, maximum expansion is set.

Preferably, the device comprises a plurality of stops positioned to ensure stepped dilation of the tubular anatomy. The stops may comprise a plurality of grooves positioned along the length of the active element and a moveable member in the form of a ring or disc. The grooves are adapted to retain the moveable ring and are each collated with a predetermined movement of the elongate members.

Alternatively, the device comprises a screw thread extending along the length of the active element, and a moveable member, moveable along the screw thread.

Conveniently, the moveable member is internally threaded.

The moveable member is moveable along the screw thread. This means that the movement of the elongate members is not limited to predetermined positions defined by the position of grooves, for example. This in turn means that dilation of the active element is not stepped.

Conveniently the first and second connectors are substantially disc shaped. The device in its un-bowed state is therefore substantially cylindrical in shape. This ensures easy insertion into a tubular anatomy.

The connectors may be formed integrally with the elongate members. In other words, the elongate members and the retaining means may be formed from a unitary sheet. Alternatively, the retaining means may be formed separately from the elongate members.

Advantageously, the compressor comprises a first sleeve associated with the active element, which sleeve is mechanically driven. When activated, the sleeve pushes against one end of the active element causing compression of the active element.

Alternatively, the compressor comprises means for purling one connector towards the other. Conveniently, the compressor comprises a wire connected to the distal end of the active element. By pulling the wire it is possible to cause the distal end to move towards the other end of the active element.

Conveniently, the compressor comprises a threaded elongate member associated with the active element

Advantageously, the device further comprises an outer sleeve extending over the active element This allows smooth passage of the device through the tubular anatomy. The outer sleeve may be made from any suitable material, but preferably it is made from rubber.

Alternatively, the outer sleeve may be made from polytetrafluoroethylene or a polyurethane elastomer.

Other parts of the device, such as the flexible members may be made from any suitable material such as Nitinol (Nytinol).

Preferably, the device further comprises a cone at the distal end of the active element. The cone facilitates initial passage though a stricture and reduces or prevents damage to the surrounding tissue and structure of the tubular anatomy.

Conveniently, the cone is made from rubber.

The invention will now be further described by way of example only with reference to the accompanying drawings in which:
Figure 1 is a schematic representation of a device according to the present invention;
Figure 2 is a cross-sectional representation of the device of Figure 1;
Figure 3 is a schematic representation of the device of Figure 1 with the outer sleeve in place;
Figure 4 is a schematic representation of the device of Figure 1 in a non bowed stated;
Figure 5 is a schematic representation of the device of Figure 1 in a bowed state;
Figures 6 and 7 are schematic representations of a second embodiment of a device according to the present invention;
Figures 8 and 9 are schematic representations of a third embodiment of a device according to the present invention; and
Figure 10 is an illustration of components forming a plurality of devices according to an embodiment of the invention.

Referring to the figures a device according to the present invention is designated generally by the reference numeral 10. The device can be used in, for example, an oesophageal manometry and also for the measurement of dilation within a tubular anatomy such as the oesophagus. The device may also be used to dilate strictures within the oesophagus.

The invention will be described in terms of use within oesophagus of a human or animal. However, it would also be a great benefit in a wide range of surgical procedures of tubular anatomy including the oesophagus;ureter, urethra, bronchus, or similar tubular structures; and vascular and cardiac structures.

The invention may also be used in balloon angioplasty procedures.

The device 10 comprises a plurality of elongate members 12 in the form of flexible strips. The elongate members 12 may be made from any convenient material for example metal. The flexible strips are held by connectors 14, 16 which, in the non active state hold the elongate members 12 in a substantially cylindrical shape. The connectors 14, 16 together with the strips 12 comprise the active element 100 of the device 10.

Although in this example the connectors 14, 16 are shown as being formed separately from the elongate members 12, in other embodiments, the connectors 14, 16 may be formed integrally with the elongate members 12.

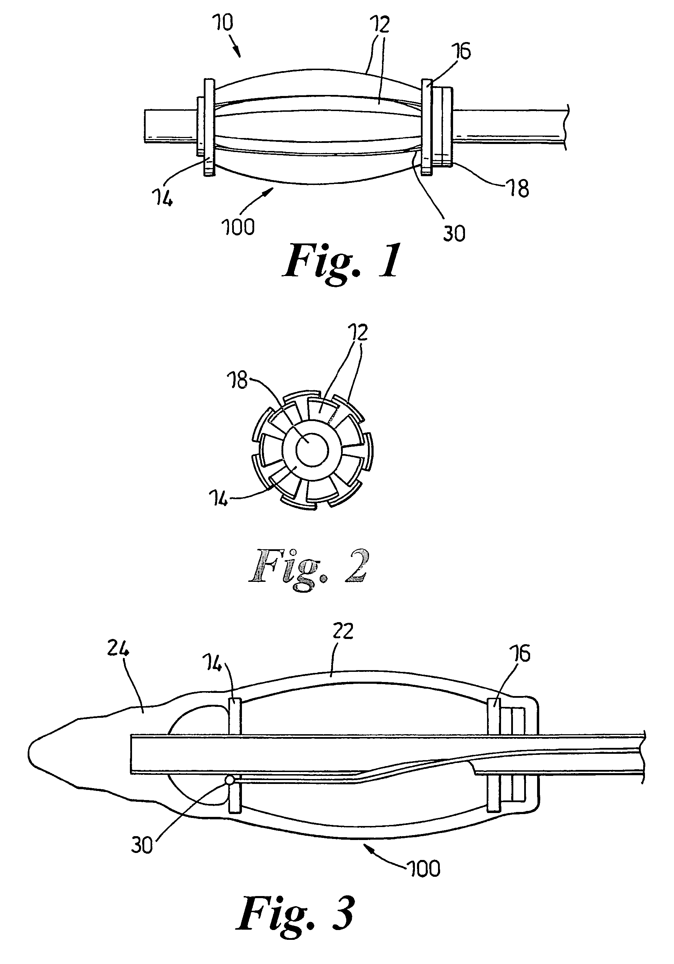

The device further comprises a first sleeve 18 which is mechanically driven. By driving the sleeve 18 towards connector 14, the elongate members 12 are caused to bow out laterally as shown in Figure 1. The sleeve 18 may be controlled by any suitable means for example it may be computer controlled.

By means of the expansion of the elongate members 12, predetermined stages of dilation of the oesophagus, particularly a stricture in the oesophagus may be achieved.

By means of the present invention therefore it is not necessary to have a range of devices of graduated size and to repeatedly insert appropriately sized devices into a patient's oesophagus. It is merely necessary to insert the device according to the invention once, and to cause dilation of the stricture in a controlled manner.

The expansion of the elongate members 12 may take place either continuously with a gradual increase in expansion, or may take place in stepped increases, depending on the conditions prevailing.

The connector 16 further comprises pressure sensors (not shown). The pressure sensors are able to measure pressure against the expanded active element. The pressure sensors are variably calibrated and are mounted proximal to a distal tip of the connector 16. The pressure sensors are able to measure proximal or distal pressure against the elongate members.

The sleeve 18 may be moved by the tensioning or pulling of an integral wire 30 connected to the distal end of the connector 14. Alternatively, a wire may be connected to the connector 16 (Figure 3).

The active element 100 is covered with an outer sleeve 22 (Figure 3) which allows smooth passage of the device 10 through an oesophagus or similar tubular anatomy.

The device further comprises a cone 24 fitted to the distal end of the device which facilitates initial passage through a stricture and prevents or reduces damage to surrounding tissue and structure. The cone may be made of soft rubber, or any other suitable material.

The device 10 is designed so that it can either form a component of an existing device, such as a flexible fibre optic endoscope, an endoscope insertion tube, a non-viewing endoscope tube; or it may be a stand alone device.

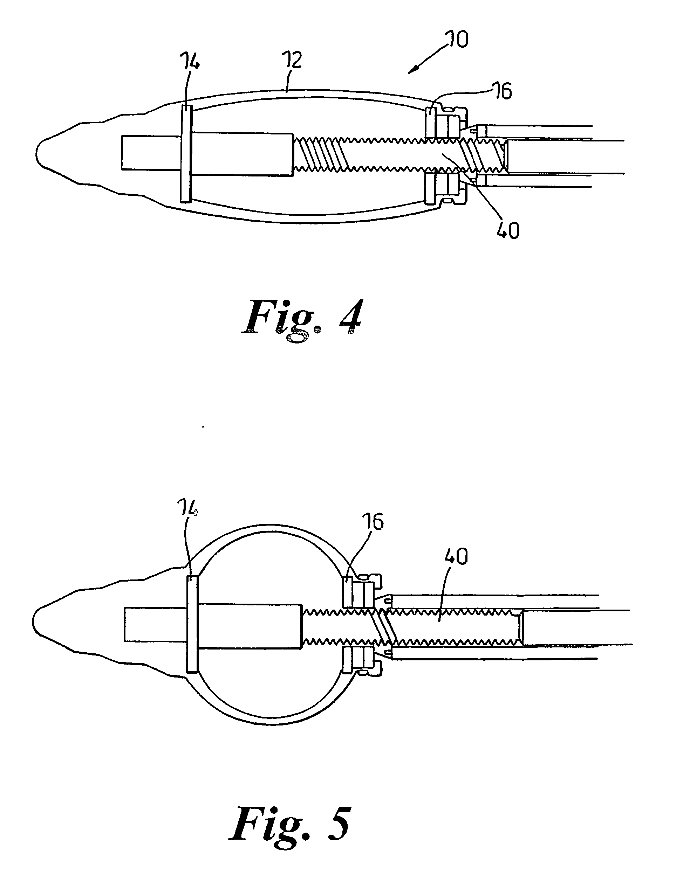

Referring to Figures 4 and 5, the device 10 is shown to comprise a compressor 40 in a form of a mechanical compression screw. The connector 14 is attached to the screw 40, and by tightening the screw 40, the connector 14 is moved towards the connector 16. This causes the elongate members 12 to bow and to take the position shown in Figure 5.

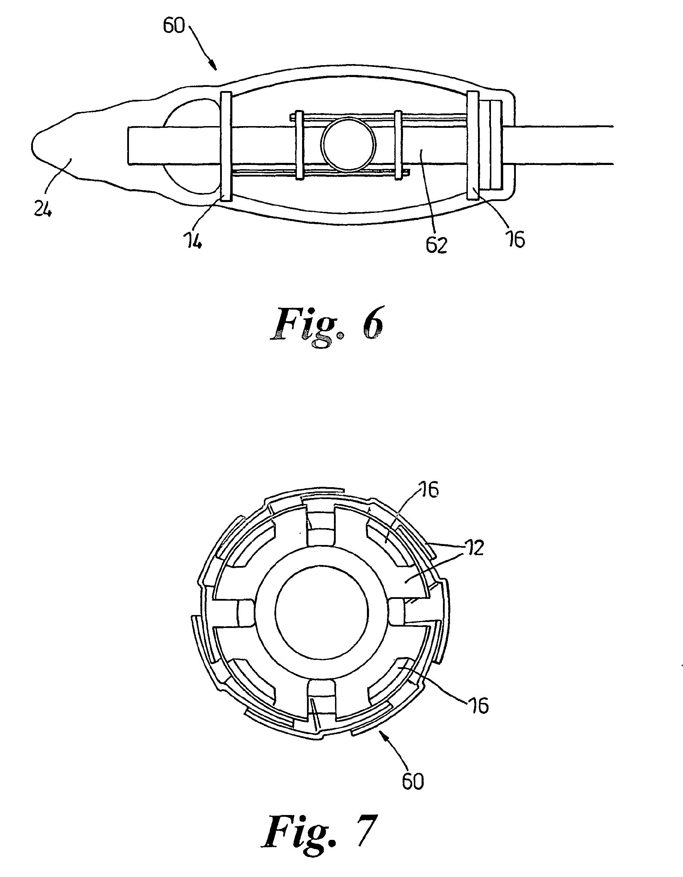

Referring now to Figures 6 and 7 a second embodiment of the present invention is shown. Parts of the device 60 which correspond to parts of the device shown in Figures 1 to 5 have been given corresponding reference numerals for ease of reference.

The device is designated generally by the reference numeral 60. The device comprises a strain sensor 62, for example a mechanical strain gauge or a peizo effect measurement-based strain sensor. The latter may be a PVDF strain sensor, essentially a silver screen printed onto clear PVDF film available from Measurement Specialities Inc (www.measurementspecialities.com). The strain gauge 62 serves to connect together the connectors 14 and 16. The gauge 62 thus replaces the mechanical compression screw 40 shown in Figures 4 and 5 and serves to cause bowing and unbowing of the device 60.

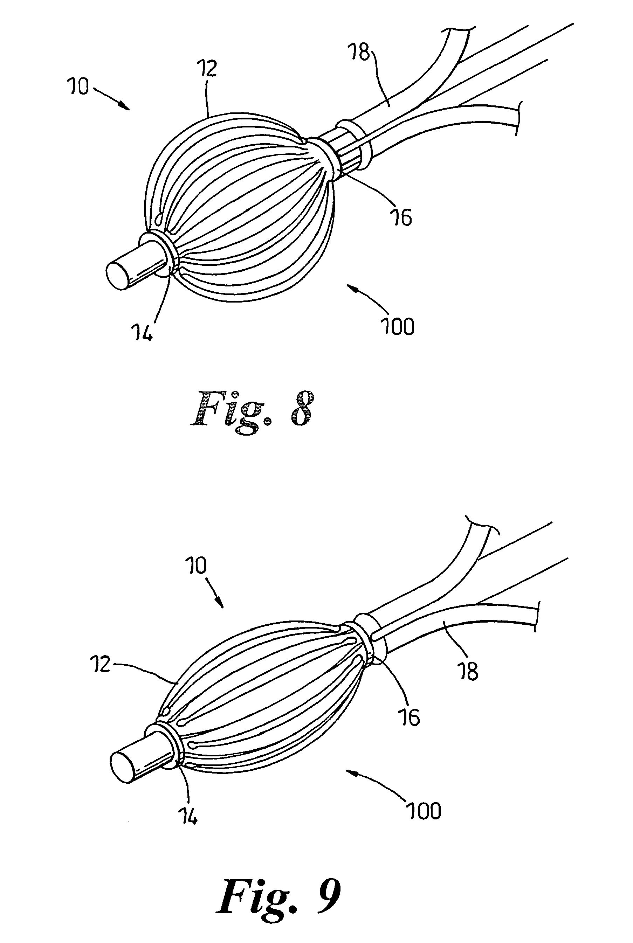

Referring to Figures 8 and 9, a further embodiment of a device 10 according to the present invention is shown. Parts of the device 10 which correspond to parts of the device 10 as illustrated in Figures 1 to 5 have been given corresponding reference numerals for ease of reference.

Figure 8 shows the device 10 in an active, or dilated state, and Figure 9 shows the device in a non-active state.

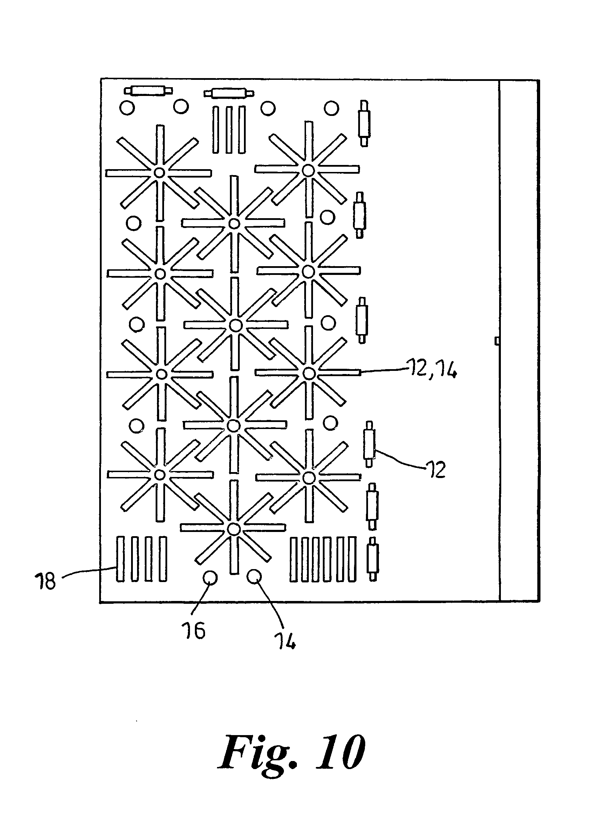

Referring to Figure 10, components which may be used to form a plurality of the devices shown in Figures 8 and 9 are illustrated. The illustrated components comprise a plurality of flexible members 12, sleeves 14, 16 and sleeves 18.

## Claims

1. A device (10) for causing dilation of, and for measuring the pressure exerted by, a tubular anatomy comprising;
an active elements (100) comprising
a plurality of elongate flexible members (12), each having a first end and a second end;
a first connector (14) connected to the first ends of the elongate flexible members;
a second connector (16) connected to the second ends of the elongate flexible members; and
a compressor, (30,40) for causing one of the connectors to move towards the other connector thereby causing the elongate flexible members to bow laterally,
**characterised in that** the first connector includes at least one pressure sensor to measure the pressure exerted on the active element by the tubular anatomy.

2. A device (10) according to Claim 1 wherein the compressor exerts a constant pressure on the active element

3. A device (10) according to Claim 1 wherein the compressor exerts stepped pressure on the active element

4. A device (10) according to any one of the preceding claims further comprising at least one stop for preventing over compression of the active element

5. A device (10) according to Claim 4 comprising a plurality of stops.

6. A device (10) according to Claim 5 wherein the stops comprise a plurality of grooves positioned along the length of the active element, and a moveable member in the form of a ring or disc.

7. A device (10) according to any one of Claims 1 to 4 further comprising a screw thread extending along the length of the active element, and a moveable member moveable along the screw thread.

8. A device (10) according to any one of the preceding claims wherein the fist and second connectors are substantially disc shaped.

9. A device (10) according to any one of the preceding claims wherein, at least one of the connectors is formed integrally with the elongate members.

10. A device (10) according to any one of Claims 1 to 8 wherein at least one of the connectors is formed separately from the elongate members.

11. A device (10) according to any one of the preceding claims further comprising a first sleeve (18) associated with the active element, which sleeve is mechanically driven.

12. A device (10) according to any one of the preceding claims wherein the compressor comprises means for pulling one connector towards the other.

13. A device (10) according to any one of the preceding claims wherein the compressor comprises a wire (30) connected to the distal end of the active element.

14. A device (10) according to any one of Claims 1 to 12 wherein the compressor comprises a screw (40).

15. A device (10) acrording to any one of Claims 1 to 12 wherein the compressor comprises a threaded elongate member associated with the elongate member.

16. A device (10) according to any one of Claims 1 to 12 wherein the compressor comprises a PVDF strain sensor (62) or strain gauge.

17. A device (10) according to any one of the preceding claims further comprising an outer sleever (22) extending over the active element.

18. A device (10) according to any one of the preceding claims further comprising a cone (24) formed at the distal end of the active element,

## Patentansprüche

1. Vorrichtung (10), um eine Dehnung einer röhrenförmigen Anatomie zu verursachen und den durch diese Anatomie ausgeübten Druck zu messen, umfassend:
ein aktives Element (100), umfassend
mehrere längliche biegsame Elemente (12), die jeweils ein erstes Ende und ein zweites Ende aufweisen;
ein erstes Verbindungsstück (14), das mit den ersten Enden der länglichen biegsamen Elemente verbunden ist; und
ein zweites Verbindungsstück (16), das mit den zweiten Enden der länglichen biegsamen Elemente verbunden ist; und
einen Kompressor (30, 40), um zu verursachen, dass sich eines der Verbindungsstücke zum anderen Verbindungsstück hin bewegt, wodurch verursacht wird, dass sich die länglichen biegsamen Elemente seitlich biegen,
**dadurch gekennzeichnet, dass** das erste Verbindungsstück zumindest einen Drucksensor umfasst, um den Druck, der durch die röhrenförmige Anatomie auf das aktive Element ausgeübt wird, zu messen.

2. Vorrichtung (10) nach Anspruch 1, wobei der Kompressor einen konstanten Druck auf das aktive Element ausübt.

3. Vorrichtung (10) nach Anspruch 1, wobei der Kompressor einen abgestuften Druck auf das aktive Element ausübt.

4. Vorrichtung (10) nach einem der vorhergehenden Ansprüche, ferner umfassend zumindest einen Anschlag, um eine Überkompression des aktiven Elements zu verhindern.

5. Vorrichtung (10) nach Anspruch 4, umfassend mehrere Anschläge.

6. Vorrichtung (10) nach Anspruch 5, wobei die Anschläge mehrere Vertiefungen, die entlang der Länge des aktiven Elements positioniert sind, und ein bewegliches Element in der Form eines Rings oder einer Scheibe umfassen.

7. Vorrichtung (10) nach einem der Ansprüche 1 bis 4, ferner umfassend ein Schraubgewinde, das sich entlang der Länge des aktiven Elements erstreckt, und ein bewegliches Element, das entlang des Schraubgewindes beweglich ist.

8. Vorrichtung (10) nach einem der vorhergehenden Ansprüche, wobei das erste und das zweite Verbindungsstück im Wesentlichen scheibenförmig sind.

9. Vorrichtung (10) nach einem der vorhergehenden Ansprüche, wobei zumindest eines der Verbindungsstücke mit den länglichen Elementen einstückig ausgeführt ist.

10. Vorrichtung (10) nach einem der Ansprüche 1 bis 8, wobei zumindest eines der Verbindungstücke von den länglichen Elementen gesondert gebildet ist.

11. Vorrichtung (10) nach einem der vorhergehenden Ansprüche, ferner umfassend eine erste Manschette (18), die dem aktiven Element zugehörig ist, welche Manschette mechanisch angetrieben wird.

12. Vorrichtung (10) nach einem der vorhergehenden Ansprüche, wobei der Kompressor Mittel umfasst, um ein Verbindungsstück zum anderen hin zu ziehen.

13. Vorrichtung (10) nach einem der vorhergehenden Ansprüche, wobei der Kompressor einen Draht (30) umfasst, der mit dem distalen Ende des aktiven Elements verbunden ist.

14. Vorrichtung (10) nach einem der Ansprüche 1 bis 12, wobei der Kompressor eine Schnecke (40) umfasst.

15. Vorrichtung (10) nach einem der Ansprüche 1 bis 12, wobei der Kompressor ein mit einem Gewinde versehenes längliches Element umfasst, das dem länglichen Element zugehörig ist.

16. Vorrichtung (10) nach einem der Ansprüche 1 bis 12, wobei der Kompressor einen Dehnungssensor (62) oder einen Dehnungsmessstreifen aus PVDF umfasst.

17. Vorrichtung (10) nach einem der vorhergehenden Ansprüche, ferner umfassend eine äußere Hülse (22), die sich über das aktive Element erstreckt.

18. Vorrichtung (10) nach einem der vorhergehenden Ansprüche, ferner umfassend einen Konus (24), der am distalen Ende des aktiven Elements gebildet ist.

## Revendications

1. Dispositif (10) pour provoquer la dilatation d'une anatomie tubulaire et pour mesurer la pression exercée par celle-ci, comprenant
un élément actif (100) comprenant
une pluralité d'éléments flexibles allongés (12) ayant chacun une première extrémité et une seconde extrémité ;
un premier raccord (14) raccordé aux premières extrémités des éléments flexibles allongés ;
un second raccord (16) raccordé aux secondes extrémités des éléments flexibles allongés ; et
un compresseur (30 ; 40) pour provoquer le déplacement de l'un des connecteurs vers l'autre connecteur, provoquant ainsi une flexion latérale des éléments flexibles allongés,
**caractérisé en ce que** le premier raccord comprend au moins un capteur de pression pour mesurer la pression exercée sur l'élément actif par l'anatomie tubulaire.

2. Dispositif (10) selon la revendication 1, dans lequel le compresseur exerce une pression constante sur l'élément actif.

3. Dispositif (10) selon la revendication 1, dans lequel le compresseur exerce une pression par paliers sur l'élément actif.

4. Dispositif (10) selon l'une quelconque des revendications précédentes, comprenant en outre au moins une butée pour empêcher une compression excessive de l'élément actif.

5. Dispositif (10) selon la revendication 4, comprenant une pluralité de butées.

6. Dispositif (10) selon la revendication 5, dans lequel les butées comprennent une pluralité de rainures positionnées sur la longueur de l'élément actif, et un élément mobile sous la forme d'un anneau ou d'un disque.

7. Dispositif (10) selon l'une quelconque des revendications 1 à 4, comprenant en outre un filetage de vis s'étendant sur la longueur de l'élément actif, et un élément mobile pouvant se déplacer le long du filetage de vis.

8. Dispositif (10) selon l'une quelconque des revendications précédentes, dans lequel les premier et second raccords ont sensiblement une forme de disque.

9. Dispositif (10) selon l'une quelconque des revendications précédentes, dans lequel au moins l'un des raccords est formé solidairement avec les éléments allongés.

10. Dispositif (10) selon l'une quelconque des revendications 1 à 8, dans lequel au moins l'un des raccords est formé séparément des éléments allongés.

11. Dispositif (10) selon l'une quelconque des revendications précédentes, comprenant en outre un premier manchon (18) associé à l'élément actif, le manchon étant entraîné mécaniquement.

12. Dispositif (10) selon l'une quelconque des revendications précédentes, dans lequel le compresseur comprend des moyens de traction d'un raccord vers l'autre.

13. Dispositif (10) selon l'une quelconque des revendications précédentes, dans lequel le compresseur comprend un fil (30) raccordé à l'extrémité distale de l'élément actif.

14. Dispositif (10) selon l'une quelconque des revendications 1 à 12, dans lequel le compresseur comprend une vis (40).

15. Dispositif (10) selon l'une quelconque des revendications 1 à 12, dans lequel le compresseur comprend un élément allongé fileté associé à l'élément allongé.

16. Dispositif (10) selon l'une quelconque des revendications 1 à 12, dans lequel le compresseur comprend un capteur de déformation en PVDF (62) ou extensomètre.

17. Dispositif (10) selon l'une quelconque des revendications précédentes, comprenant en outre un manchon externe (22) s'étendant sur l'élément actif.

18. Dispositif (10) selon l'une quelconque des revendications précédentes, comprenant en outre un cône (24) formé à l'extrémité distale de l'élément actif.
